# EUROPÄISCHE PATENTANMELDUNG

(11) **EP 0 995 420 A2**
(43) Veröffentlichungstag der Anmeldung: **26.04.2000**
(21) Anmeldenummer: 99107748.8
(22) Anmeldetag: 19.04.1999
(51) Int. Cl.: A61K 6/00

(54) **Zahnprothesen-Haftmittel, insbesondere für den Unterkieferbereich**

(30) Priorität: 23.07.1998 AT 126798
(71) Anmelder: Altwirth, Oskar, A-4950 Altheim (AT)
(72) Erfinder: Altwirth, Oskar, A-4950 Altheim (AT)

(57) **Zusammenfassung**

Es wird ein Haftmittel für Zahnprothesen, insbesondere für den Unterkieferbereich, mit einer pastösen Trägersubstanz, in die Hydrokolloide eingemengt sind, sowie ein Verfahren zu seiner Herstellung beschrieben. Die Trägersubstanz dieses Haftmittels besteht aus einer alkoholischen Lösung eines Glycerinresinates mit Beimengungen von Propolis.

## Beschreibung

Die Erfindung bezieht sich auf ein Haftmittel für Zahnprothesen, insbesondere für den Unterkieferbereich, mit einer pastösen Trägersubstanz, in die Hydrokolloide eingemengt sind, sowie auf ein Verfahren zur Herstellung eines solchen Haftmittels.

Übliche Haftmittel für Zahnprothesen weisen eine pastenförmige Trägersubstanzen, wie Petrolatum, auf und werden nach dem Gebrauch der Zahnprothese wieder von der Zahnprothese abgespült. Nachteilig bei diesen Haftmitteln ist vor allem, daß sie durch das Eindringen von Speichel Immer weicher und am Ende ausgespült werden, wodurch die Haftfähigkeit verloren geht. Um die Haftwirkung zu verlängern, werden Haftmittel auf der Basis von von Polyvinylacetat eingesetzt. Trotz der Verwendung von Polyvinylacetat bleibt jedoch die Haftwirkung zeitlich beschränkt, weil die eingemengten Hydrokolloide mit der Zeit ausgeschwemmt werden und dadurch die Stabilität des Polyvinylacetates verringert wird. Es besteht daher der Bedarf nach einem gesundheitlich unbedenklichen Haftmittel für Zahnprothesen, das über die übliche tägliche Tragezeit einer Zahnprothese einen sicheren Halt gewährleistet, und zwar auch bei schwierigen Kieferverhältnissen.

Der Erfindung liegt daher die Aufgabe zugrunde, ein Haftmittel für Zahnprothesen der eingangs geschilderten Art zu schaffen, das kaum ausschwemmbar ist und eine hohe Stabilität der Trägersubstanz auch über längere Zeiträume sicherstellt.

Die Erfindung löst die gestellte Aufgabe dadurch, daß die Trägersubstanz aus einer alkoholischen Lösung eines Glycerinresinates mit Beimengungen von Propolis besteht.

Da das eingesetzte Glycerinresinat, ein Naturharz, die Fähigkeit besitzt, sich in gelöstem Zustand mit Hydrokolloiden innigst zu verbinden, wird ein vom Speichel nicht lösbares Haftmittel erhalten, das unter der Zahnprothese nicht erweicht, sondern erhärtet. Diese vorteilhafte, für die erfindungsgemäße Wirkung wesentliche, innige Verbindung der Hydrokolloide mit dem verwendeten Glycerinresinat gelingt jedoch nur, wenn zugleich Propolis, also ein Vorwachs der Bienen eingesetzt wird, dessen Hauptbestandteile aus Palmitinsäuremyricilester, Cerotinsäure und kohlenwasserstoffen bestehen. Mit Hilfe von Propolis läßt sich nämlich das sonst in Alkohol nicht lösliche Glycerinresinat in Äthylalkohol lösen, was eine Voraussetzung für die Verbindung mit den Hydrokolloiden ist. Mit Hilfe eines erfindungsgemäßen Haftmittels, das gesundheitlich unbedenklich ist, weil seine Bestandteile auch im Lebensmittelbereich eingesetzt werden, kann eine gute, dauerhafte Klebewirkung erzielt werden, und zwar nicht nur im Bereich des Zahnfleisches, sondern auch im Prothesenbereich, so daß sich ein solches Haftmittel insbesondere für den Unterkieferbereich anbietet. In diesem Zusammenhang ist zu bedenken, daß sich durch das Erhärten des Haftmittels unter der Zahnprothese ein Unterdruck ausbilden kann, der die Haftfähigkeit unterstützt. Wird ein unter dem Namen "Ester Gum" bekannter Ester der Abietinsäure als Glycerinresinat verwendet, so können besonders günstige Verhältnisse erreicht werden.

Die molekulare Beschaffenheit der Hydrokolloide ist für das Einbringen dieser Hydrokolloide in das Glycerinresinat von nicht unerheblicher Bedeutung. Der Einsatz von Natriumcarboxymethylcellulose oder einem Polysaccharid bzw. einem Natriumalginat haben sich in diesem Zusammenhang besonders bewährt.

Um das Glycerinresinat in eine alkoholische Lösung überführen zu können, wird ein Verfahren vorgeschlagen, bei dem zunächst eine Lösung aus Äthylalkohol und Propolis hergestellt wird, bevor dieser Lösung zuerst das Glycerinresinat und dann die Hydrokolloide beigemengt werden. Durch das vorausgehende Lösen von Propolis in Äthylalkohol können in überraschender Weise die Voraussetzungen für das Lösen von Glycerinresinat geschaffen werden. Das Petrolatum wird dabei zur Einstellung der pastösen Eigenschaften des späteren Haftmittels benötigt. Dem in dieser Weise gelösten Glycerinresinat können dann die Hydrokolloide ohne weiteres beigemengt werden.

Im einzelnen kann dabei so vorgegangen werden, daß 8 bis 12 Gewichtsteile Propolis in 92 bis 88 Gewichtsteilen Äthylalkohol gelöst und 100 Gewichtsteilen dieser Lösung 5 bis 15 Gewichtsteile Petrolatum bei etwa 140 °C zugemischt werden und daß dann bei annähernd gleicher Temperatur in 100 Gewichsteilen dieser Vormischung 65 bis 75 Gewichtsteile Glycerinresinat gleichmäßig eingerührt werden, bevor nach einer Abkühlung auf ca. 30 °C 100 Gewichtsteilen dieses Zwischenproduktes 130 bis 150 Gewichtsteile an Natriumcarboxymethylcellulose oder einem Polysaccharid bzw. einem Natriumalginat beigemengt werden.

### Beispiel:

Unter Erwärmung von 90 Gewichtsteilen Äthylalkohol werden 10 Gewichtsteile Propolis gelöst, wobei die Erwärmung in einem geschlossenen Rührwerk erfolgt, um eine Verflüchtigung des Alkohols zu verhindern. Außerdem ist der Alkoholstand durch eine entsprechende Ergänzung konstant zu halten. In die gewonnene Lösung wird im Verhältnis von 10 Gewichtsteilen dieser Lösung ein Gewichtsteil Petrolatum eingerührt. Der Rührvorgang dauert etwa 10 Minuten und wird bei einer Temperatur von ca. 140 °C durchgeführt. Bei gleicher Temperatur werden im Verhältnis von 10 Gewichtsteilen der bisherigen Vormischung 7 Gewichtsteile Ester Gum durch gleichmäßiges langsames Verrühren mit besonderen engen Rührflügeln zugemischt. Nach etwa 30 Minuten wird die Mischung auf 30 °C abgekühlt. Dann werden je 100 Gewichtsteilen dieses Zwischenproduktes 144 Gewichtsteile eines Hydrokolloides, nämlich Natriumcarboxymethylcellulose oder ein Polysaccharid bzw. einem Natriumalginat beigemengt, wobei zur Erreichung des Endproduktes der Mischvorgang abhängig von der Masse 60 - 120 Minuten dauert.

## Patentansprüche

1. Haftmittel für Zahnprothesen, insbesondere für den Unterkieferbereich, mit einer pastösen Trägersubstanz, in die Hydrokolloide eingemengt sind, dadurch gekennzeichnet, daß die Trägersubstanz aus einer alkoholischen Lösung eines Glycerinresinates mit Beimengungen von Propolis besteht.

2. Haftmittel nach Anspruch 1, dadurch gekennzeichnet, daß das Glycerinresinat aus einem Ester der Abietinsäure besteht.

3. Haftmittel nach Anspruch 1 oder 2, dadurch gekennzeichnet, daß die Hydrokolloide aus Natriumcarboxymethylcellulose oder einem Polysaccharid bzw. einem Natriumalginat bestehen.

4. Verfahren zur Herstellung eines Haftmittels für Zahnprothesen nach einem der Ansprüche 1 bis 3, dadurch gekennzeichnet, daß zunächst eine Lösung aus Äthylalkohol und Propolis hergestellt wird, bevor dieser Lösung zuerst Glycerinresinat und dann die Hydrokolloide beigemengt werden.

5. Verfahren nach Anspruch 4, dadurch gekennzeichnet, daß 8 bis 12 Gewichtsteile Propolis in 92 bis 88 Gewichtsteilen Äthylalkohol gelöst und 100 Gewichtsteilen dieser Lösung 5 bis 15 Gewichtsteile Petrolatum bei etwa 140 °C zugemischt werden und daß dann bei annähernd gleicher Temperatur in 100 Gewichsteilen dieser Vormischung 65 bis 75 Gewichtsteile Glycerinresinat gleichmäßig eingerührt werden, bevor nach einer Abkühlung auf ca. 30 °C 100 Gewichtsteilen dieses Zwischenproduktes 130 bis 150 Gewichtsteile an Natriumcarboxymethylcellulose oder einem Polysaccharid bzw. einem Natriumalginat beigemengt werden.
